# EUROPÄISCHE PATENTANMELDUNG

(11) **EP 1 138 667 A1**
(43) Veröffentlichungstag der Anmeldung: **04.10.2001**
(21) Anmeldenummer: 00123845.0
(22) Anmeldetag: 02.11.2000
(51) Int. Cl.: C07C 255/46, C07C 253/10

(54) **Verfahren zur Herstellung von Cyclopentanonnitrilen**

(30) Priorität: 25.03.2000 DE 10015063
(71) Anmelder: Degussa AG, 40474 Düsseldorf (DE)
(72) Erfinder: De Schrijver, Johny, 9111 Sint-Niklaas (BE)

(57) **Zusammenfassung**

Die vorliegende Erfindung ist auf ein Verfahren zur Herstellung von Verbindungen der allgemeinen Formel (I) 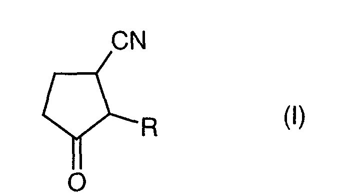 worin R einen linearen oder beliebig verzweigten (C₁-C₁₈)-Alkylrest darstellt, gerichtet.

Dabei geht man von Verbindungen der allgemeinen Formel (II) 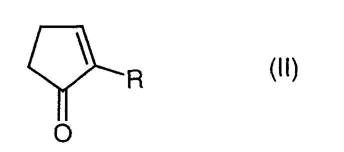 worin R die oben angegebene Bedeutung inne hat, aus, welche in Gegenwart von Blausäure oder eine die Blausäure in situ produzierende Vorstufe und katalytischen Mengen an Base umgesetzt werden. Dabei ist die Anwesenheit eines weiteren polaren Lösungsmittels nicht erforderlich.Verbindungen der Formel (I) sind wichtige Intermediate für Riechstoffe und Aromen.

## Beschreibung

Die vorliegende Erfindung bezieht sich auf ein Verfahren zur Herstellung von Verbindungen der allgemeinen Formel (I).

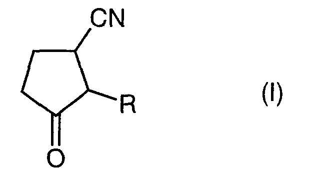

Diese Nitrile sind wertvolle Intermediate für die Herstellung von Riechstoffen und Aromen.

Laut US 4,016,109 werden derartige Verbindungen entweder unter sauren Bedingungen durch Michael-Addition von Cyanid-Ionen oder durch basenkatalysierte Addition von Blausäure, welche aus Acetoncyanhydrin in situ freigesetzt wird, an das entsprechende Cyclopentenon gewonnen. Letztere Methode hat zum einen den Nachteil, daß die Blausäure erst in das Cyanhydrin umgewandelt werden und aus diesem anschließend wieder freigesetzt werden muß. Außerdem ist bei dieser Reaktion ein organisches Lösungsmittel anwesend. Bei großtechnischen Verfahren ist es hingegen von Vorteil, wenn man kostengünstig arbeiten kann, was u.a. heißt, möglichst wenig Einsatzstoffe bei den Verfahren einzusetzen.

Aufgabe der vorliegenden Erfindung war es deshalb, ein weiteres Verfahren zur Herstellung der Verbindungen der allgemeinen Formel (I) anzugeben, welches insbesondere die Herstellung von (I) in kostengünstigerer Weise erlaubt.

Diese und andere nicht näher spezifizierte Aufgaben, welche sich jedoch ohne Umstand aus dem Stand der Technik ergeben, werden durch ein Verfahren mit den kennzeichnenden Merkmalen des Anspruchs 1 gelöst. Ansprüche 2 bis 4 beziehen sich auf besondere Ausgestaltungen des erfindungsgemäßen Verfahrens. Anspruch 5 schützt eine besondere Verwendung.

Dadurch,daß man für die Herstellung von Verbindungen der allgemeinen Formel (I) 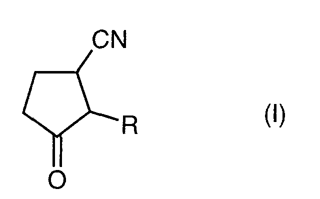 worin R einen linearen oder beliebig verzweigten (C₁-C₁₈)-Alkylrest darstellt, von Verbindungen der allgemeinen Formel (II) 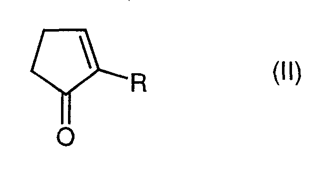 worin R die oben angegebene Bedeutung inne hat, ausgeht und diese in Gegenwart von Blausäure oder einer die Blausäure in situ produzierenden Vorstufe und katalytischen Mengen Base umsetzt, gelingt es völlig überraschend, die Reaktion in Abwesenheit eines weiteren organischen Lösungsmittels durchzuführen.

Dies lag mithin keineswegs nahe, da es sich bei den eingesetzten Basen auch um salzartige Verbindungen handelt, welche normalerweise nur in sehr polaren organischen Lösungsmitteln, wie z.B. Methanol, löslich sind und die Gefahr bestand, daß diese Stoffe durch Ausfallen der Reaktion entzogen werden und keine Wirkung mehr entfalten können. So wird beispielsweise in der DE 3942371 und DE 10085871 bei vergleichbarer Reaktion die Anwesenheit von polaren organischen Lösungsmitteln (DMF oder Überschuß an Isophoron) gerade bevorzugt.
Unter den gegebenen Umständen ist es deshalb als überraschend zu werten, daß unter diesen Bedingungen keine größe-ren Nebenreaktionen, wie z.B Aldol oder Michael-Addition von Enon (II) und Nitril (I) oder Polymerisation der Blausäure, stattfinden. Es wird sogar völlig überraschend mit den erfindungsgemäßen Reaktionsbedingungen eine verglichen mit dem Stand der Technik bessere Ausbeute von > 94% an Nitril (I) erzielt.

Als einzusetzende Basen kommen im Prinzip alle dem Fachmann für diesen Zweck geläufigen organischen und anorganischen Basen in Betracht, sofern sie ausreichende Wirkung für die Katalyse der Michael-Addition zeigen. Dieses ist in Routi-neexperimenten leicht zu klären. Beispielhaft sind die in DE 3942371 genannten anzuführen.
Vorzugsweise verwendet man als Basen günstige anorganische Stoffe wie z.B. NaOH, Na₂CO₃, Ca(OH)₂ etc. Besonders bevorzugt wird LiOH verwendet.

Die Menge der einzusetzenden Base richtet sich nach deren Wirksamkeit im Reaktionsmedium und der Möglichkeit Stoff-einsatzkosten zu minimieren. Bevorzugt setzt man die Base in einer Menge von 0,01 bis 10 Mol%, vorzugsweise 0,1 bis 5 Mol%, besonders bevorzugt 0,5 bis 5 Mol%, bezogen auf das Enon (II) ein.

Die Temperatur kann bei der ins Auge gefaßten Reaktion beliebig gewählt werden. Sie sollte so bemessen sein, daß die Reaktion möglichst schnell abläuft, daß auf der anderen Seite aber möglichst wenig Nebenprodukte gebildet werden. Vorteilhaft arbeitet man bei einer Temperatur von 10-120 °C, vorzugsweise 20-100 °C, besonders bevorzugt 30-80 °C.

In einer weiteren Ausgestaltung befaßt sich die vorliegende Erfindung mit der Verwendung des erfindungsgemäß hergestellten Nitrils (I) in einem Verfahren zur Herstellung von Riechstoffen der allgemeinen Formel (III), 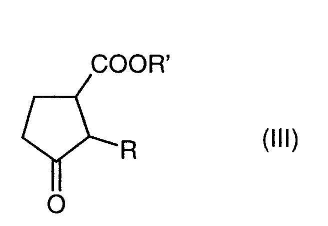 worin R die oben angegebene Bedeutung inne hat und R' = H, Na, K, Li, Ca_{1/2}, Mg_{1/2}, einen linearen oder beliebig verzweigten Rest der Gruppe (C₁-C₁₈)-Alkyl angibt, wobei man vorzugsweise das Nitril (I) nicht zwischenisoliert, sondern gleich an dessen Herstellung die Umwandlung der Nitrilfunk-tion anknüpft. Dies wird erst dadurch möglich, daß das Nitril (I) aufgrund des erfindungsgemäßen Verfahrens in einer äußerst reinen Form dargestellt werden kann und ansonsten entstehende Nebenprodukte den letztlich erhaltenen Riechstoff nicht verunreinigen. Die Weiterverarbeitung von (I) zu (III) kann analog der US 4,016,109 erfolgen.

Unter einem linearen oder beliebig verzweigten (C₁-C₁₈)-Alkylrest wird im Rahmen der Erfindung ein Alkylrest mit 1 bis 18 C-Atomen verstanden, der sämtliche theoretisch mögliche Bindungsisomere umfaßt, wie z.B. Methyl Ethyl, Propyl, Isopropyl, n-Butyl, sec-Butyl, Isobutyl, tert.-Butyl, Pentyl, Hexyl, Heptyl, Octyl, Nonyl, Decyl, Undecyl, Dodecyl usw.

Gleichfalls sind im Rahmen der Erfindung mit der Angabe der allgemeinen Formeln auch alle möglichen Enantiomere und Diastereomere sowie Mischungen derselben mitumfaßt.

### Beispiel:

Laborprozedur für die Herstellung von 2-n-Hexyl-3-cyanocyclopentanon.

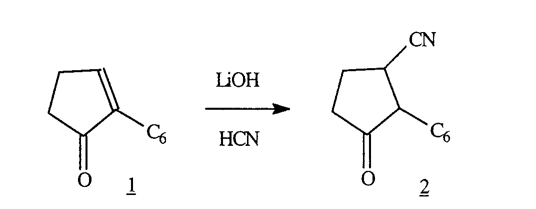

Zu 2-n-Hexylcyclopentenon (1402,8 g; 8,435 Mol) wird bei 40°C Lithiumhydroxid (4,07 g; 0,17 Mol) dosiert. Blausäure (251,6 g; 9,28 Mol, 1,1 Äq.) wird bei 40°C in 30 Minuten zudosiert, wobei die Temperatur bis auf 62°C anstiegt. Das Gemisch wird zusätzlich noch 2 Stunden bei 55°C erwärmt. Die Ausbeute an 2-n-Hexyl-3-cyanocyclopentanon beträgt 94,3% (1534,3 g).

## Patentansprüche

1. Verfahren zur Herstellung von Verbindungen der allgemeinen Formel (I) 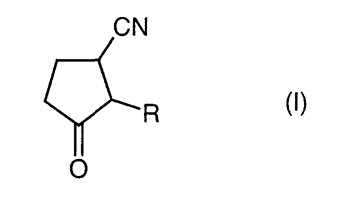 worin R einen linearen oder beliebig verzweigten (C₁-C₁₈) -Alkylrest darstellt,
aus Verbindungen der allgemeinen Formel (II) 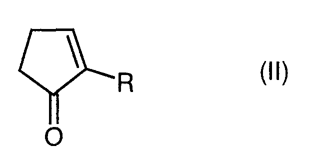 worin R die oben angegebene Bedeutung inne hat, in Gegenwart von Blausäure oder einer die Blausäure in situ produzierenden Vorstufe und katalytischen Mengen Base,
**dadurch gekennzeichnet, daß** man in Abwesenheit eines weiteren organischen Lösungsmittels arbeitet.

2. Verfahren nach Anspruch 1,
**dadurch gekennzeichnet, daß**
man als Base LiOH verwendet.

3. Verfahren nach Anspruch 1 oder 2,
**dadurch gekennzeichnet, daß**
man die Base in einer Menge von 0,01 bis 10 Mol% , vorzugsweise 0,1 bis 5 Mol%, besonders bevorzugt 0,5 bis 5 Mol%, bezogen auf das Enon (II) einsetzt.

4. Verfahren nach einem oder mehreren der vorhergehenden Ansprüche,
**dadurch gekennzeichnet, daß**
man bei einer Temperatur von 10-120 °C, vorzugsweise 20-100 °C, besonders bevorzugt 30-80 °C, arbeitet.

5. Verwendung des nach Anspruch 1 hergestellten Nitrils (I) in einem Verfahren zur Herstellung von Riechstoffen der allgemeinen Formel (III), 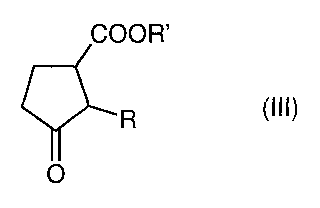 worin R die in Anspruch 1 angegebene Bedeutung inne hat und R' = H, Na, K, Li, Ca_{1/2}, Mg_{1/2}, einen linearen oder beliebig verzweigten Rest der Gruppe (C₁-C₁₈)-Alkyl darstellt.

6. Verwendung nach Anspruch 5,
**dadurch gekennzeichnet, daß**
man das Nitril (I) nicht zwischenisoliert.
